# EUROPÄISCHE PATENTSCHRIFT

(11) **EP 0 684 956 B1**
(45) Veröffentlichungstag und Bekanntmachung des Hinweises auf die Patenterteilung: **16.07.2003**
(21) Anmeldenummer: 95905572.4
(22) Anmeldetag: 21.12.1994
(51) Int. Cl.: C07K 14/765, G01N 33/531, G01N 33/569, G01N 33/576

(54) **ACYLIERTE PROTEINAGGREGATE UND DEREN VERWENDUNG ZUR SIGNALSTEIGERUNG IN EINEM IMMUNOASSAY ZUM NACHWEIS VON ANTIKÖRPERN**
ACYLATED PROTEIN AGGREGATES AND THEIR USE IN ENHANCING SIGNALS IN AN IMMUNOASSAY TESTING FOR ANTIBODIES
AGREGATS DE PROTEINES ACYLES ET LEUR UTILISATION POUR AUGMENTER LES SIGNAUX DANS UN DOSAGE IMMUNOLOGIQUE DESTINE A LA DETECTION D'ANTICORPS

(30) Priorität: 21.12.1993 DE 4343480
(43) Veröffentlichungstag der Anmeldung: 06.12.1995
(73) Patentinhaber: Roche Diagnostics GmbH, 68305 Mannheim (DE)
(72) Erfinder: SCHMITT, Urban, D-82386 Oberhausen (DE); SCHLIEPER, Dittmar, D-82393 Iffeldorf (DE); SCHMID, Franz, D-86911 Diessen (DE)
(86) Internationale Anmeldenummer: EP9404265
(87) Internationale Veröffentlichungsnummer: WO95017427

(56) Entgegenhaltungen:
- EP-A- 0 122 209
- EP-A- 0 269 092
- US-A- 5 051 356

## Beschreibung

Die Erfindung betrifft acylierte Proteinaggregate, deren Herstellung und deren Einsatz in einem Mittel und in einem Reagenz für immunologische Teste und deren Verwendung zur Signalsteigerung von Immunoassays zum Nachweis von Antikörpern sowie ein entsprechendes immunologisches Nachweisverfahren.

Immunologische Nachweismethoden haben in den letzten Jahren eine große Bedeutung erlangt. Mit ihnen kann die Gegenwart von Arzneimitteln, Hormonen, Proteinen und insbesondere infektiösen Organismen in biologischen Proben schnell und genau nachgewiesen werden. Bei allen immunologischen Nachweismethoden kommt es zu einer spezifischen Bindungsreaktion zwischen einem ersten spezifischen Bindungspartner der Substanz, die nachgewiesen werden soll ("Analyt") und einem zweiten spezifischen Bindungspartner, der spezifisch mit dem Liganden reagiert oder ihn bindet. Ligand und spezifischer Ligandbindungspartner, die sogenannten Partner eines spezifischen Bindungspaares, bilden dabei ein spezifisches Bindungspaar, im allgemeinen ein Komplex zwischen einem Antigen und einem Antikörper oder Antikörperfragment. Dabei können mehr als ein Ligand oder ein Bindungspartner in jeder Reaktion miteinander reagieren. Diese spezifischen Bindereaktionen werden auf verschiedene Weise detektiert. Im allgemeinen ist ein Teilnehmer der spezifischen Bindereaktion markiert. Übliche Markierungsmethoden sind Radioisotope, Chromogene, Fluorogene oder Enzymmarkierung. Bei heterogenen Immunoassays ist einer der Bindungspartner an eine Festphase immobilisiert.

Der Nachweis von spezifischen Antikörpern, die gegen ein Antigen gerichtet sind, in einem heterogenen Immunoassay, insbesondere einem sog. Sandwichimmunoassay wird demnach im allgemeinen so durchgeführt, daß ein an die Festphase gebundenes Antigen in einem ersten Inkubationsschritt mit der zu bestimmenden Probe, im Regelfall Humanserum bzw. Plasma, in Kontakt gebracht wird und die in der Probe enthaltenen Antikörper gegen das Antigen während einer Inkubationsdauer an das wandgebundene Antigen binden können. Nach der Reaktion wird der Testansatz gewaschen und in einem zweiten Reaktionsschritt werden die an der Festphase gebundenen Antikörper durch einen zweiten Antikbrper, der eine Markierung trägt und gegen die zu bestimmende Antikörperklasse gerichtet ist, nachgewiesen. Dieser Nachweis geschieht in der Weise, daß ein gegen die zu bestimmende Antikörperklasse gerichteter markierter Antikörper (monoklonal oder polyklonal) oder ein markiertes Analytanalogon in einem zweiten Inkubationsschritt an die Antikörper bindet, die an das wandständige Antigen im ersten Inkubationsschritt gebunden haben. Sollen IgG-Antikörper, nachgewiesen werden, verwendet man bevorzugt ein markiertes Anti-IgG, bei IgM-Antikörpern ein Anti-IgM usw Nach der zweiten Inkubation wird der Testansatz gewaschen und die nicht gebundenen überschüssigen markierten Anti-Antikörper oder Analytanaloga entfernt. In einem dritten Schritt wird die Menge an gebundenen Antikörper in der Weise detektiert, daß zum Beispiel bei Enzymimmunoassays der Testansatz mit einer Substratlösung reagiert und der gebildete Farbkomplex photometrisch gemessen wird. Die Extinktion oder das Signal dieser Farblösung ist proportional der Menge an gebundenen Antikörpern.

In einer besonderen Ausführungsform dieser Antikörper-Immunoassays ist das Antigen nicht direkt an die Festphase gebunden, sondern wird über einen weiteren spezifischen Bindungspartner vor oder bevorzugt während des Tests an die Festphase gebunden. Bevorzugt koppelt dabei ein mit Biotin kovalent gekoppeltes Antigen an eine mit Streptavidin beladene Festphase.

Eine besondere Art der Störung von Antikorpertesten wird insbesondere beim Nachweis von IgG-Antikörpern gegen ein Antigen, zum Beispiel HIV, HCV, Toxoplasmose, Antikörper usw. beobachtet. Verdünnt man gleiche Mengen einer Probe eines Humanserums, das eine hohe Konzentration eines gegen ein Antigen gerichteten Antikörpers enthält (zum Beispiel Antikörper gegen HCV), mit verschiedenen Seren, die keine Antikörper gegen dieses Antigen besitzen, so findet man signifikante Unterschiede in der Wiederfindung der Antikörpermenge in den so verdünnten Seren, obwohl in allen Seren die gleiche Gesamtmenge an spezifischen Antikörpern enthalten ist. Bei Enzymimmunoassays ist ein um den Faktor 2-10 zu kleines Signal in den mit Serum verdünnten Proben zu messen. Eine Erklärung für dieses Phänomen ist nicht bekannt, könnte eventuell aber darin liegen, daß in Humanseren Substanzen enthalten sind, d ie die Bindung spezifischer Antikorper an ein Antigen behindern. Eine andere oder zusätzliche Erklärungsmoglichkeit könnte sein, daß diese in Humanseren enthaltenen Substanzen die Detektion der gebundenen Antikörper im zweiten Inkubationsschritt behindern. Die durch Serumbestandteile bewirkten drastischen Signalreduktionen sind besonders kritisch bei schwach positiven Seren, die im ungünstigen Fall als negativ detektiert und somit falsch diagnostiziert werden

Aufgabe der vorliegenden Erfindung war es daher, eine genügend große Signalsteigerung in einem Immunoassay zum Nachweis von Antikörpern zu erreichen, so daß falsch negative Analyseergebnisse reduziert oder vermieden werden. Die Aufgabe wird gelöst durch die Verwendung von spezifisch acylierten Proteinaggregaten in Immunoassays.

Gegenstand der Erfindung sind Proteinaggregate als Substanzen zur Signalsteigerung in Immunoassays zur Bestimmung von Antikörpern, die mit -CO-R-Gruppen acyliert sind, wobei R einen verzweigten oder unverzweigten C1 - C4-Alkylrest darstellt, der mit Carboxy, Hydroxy, SO₃H oder PO₃H₂ substituiert sein kann.

Ein weiterer Gegenstand der Erfindung ist ein entsprechendes Mittel zur Steigerung des Signals bei Immunoassays enthaltend einen Puffer und eines oder mehrere der erfindungsgemäßen Substanzen zur Signalsteigerung.

Ein weiterer Gegenstand der Erfindung ist ein spezifisches Bindereagenz für Immunoassays zur Bestimmung von Antikörpern enthaltend einen Bindungspartner für den zu bestimmenden Antikörper, dadurch gekennzeichnet, daß es zusätzlich eine oder mehrere der erfindungsgemäßen Substanzen oder Mittel zur Signalsteigerung des Immunoassays enthält.

Ein weiterer Gegenstand der Erfindung ist ein Verfahren zur Signalsteigerung und zur Reduktion falsch-negativer Analyseergebnisse in Immunoassays zur Bestimmung von Antikörpern durch Inkontaktbringen einer erfindungsgemäßen Substanz oder des erfindungsgemäßen Mittels zur Signalsteigerung mit den gegen den Antikörper gerichteten spezifischen Bindungspartnern, insbesondere mit dem unmarkierten Bindungspartner.

Insbesondere ist Gegenstand der Erfindung ein Verfahren zur Bestimmung eines Antikörpers in einer Probe unter Signalsteigerung und Reduktion falsch-negativer Analyseergebnisse durch

Kontaktieren der auf den Antikörper zu untersuchender Probe mit einem oder mehreren spezifischen Bindungspartnern des Antikörpers, wobei mindestens ein Bindungspartner markiert ist und ein detektierbares Bindungspaar mit dem Antikörper bildet,

Messung des Signals des markierten Bindungspaares oder des freien markierten Bindungspartners als Maß für die Anwesenheit oder Konzentration des Antikörpers in der Probe,
dadurch gekennzeichnet, daß zur Probe oder zu einem der spezifischen Bindungspartner ein Proteinaggregat zugegeben wird, das mit -CO-R-Gruppen acyliert ist, wobei R einen verzweigten oder unverzweigten C1-C4-Alkylrest darstellt, der durch Carboxy, Hydroxy, SO₃H oder PO₃H₂ substituiert sein kann.

Als Probe dienen im allgemeinen Körperflüssigkeiten wie Blut, Serum oder Plasma, Speichel, Urin oder andere Körperflüssigkeiten, insbesondere Serum oder Plasma.

Nachzuweisende Antikörper sind alle gegen ein Antigen gerichtete Antikörper, insbesondere Immunoglobulin-Antikörper.

Als spezifischer Bindungspartner kann jeder biologische oder chemische Bindungspartner dienen, der spezifisch mit dem zu bestimmenden Antikörper zu einem spezifischen Bindungspaar reagien. Dies sind insbesondere Antigene, Haptene und Anti-Antikörper.

Mindestens einer der spezifischen Bindungspartner in einem Immunoassay ist markiert. Die Markierung kann direkt oder indirekt ein meßbares Signal liefern, zum Beispiel durch Radioaktivität, Chemilumieszenz, Elektrochemilumineszenz, Phosphoreszenz, Floureszenz oder sichtbare Farbe Der spezifische Bindungspartner kann auch indirekt detektierbar sein, zum Beispiel mit Enzymmarkierung, die in einer oder mehreren Reaktionen teilnimmt, um eine detektierbar Substanz zu erzeugen. Bevorzugt werden in der vorliegenden Erfindung Enzymmarkierung, insbesondere mit Peroxidase, Glucoseoxidase, Phosphatase oder β-Galaktosidase oder Elektrochemolumineszenz. Bevorzugt wird in der vorliegenden Erfindung ein gegen den nachzuweisenden Antikörper gerichteter markierter Anti-Antikörper eingesetzt.

Die erfindungsgemäßen Signalsteigerungssubstanzen sind acyliene Proteinaggregate. Unter einem Proteinaggregat versteht man ein Aggregat, das aus gleichen oder verschiedenen definierten Proteinmonomeren zu einem höher molekularen Partikel polymerisiert wurde. Definitionsgemäß wird dabei unter einem Proteinaggregat ein kunstliches Partikel aus mindestens 2, bevorzugt 3- 40.000, besonders bevorzugt 30 - 600 Proteinmonomeren verstanden, die so fest aneinander gebunden sind, daß sie in wäßriger Lösung nicht in die Proteineinzelmoleküle zerfallen. Bevorzugt sind die Proteinaggregate wasserlöslich.

Proteine können thermisch oder chemisch polymerisiert oder aggregiert werden.

Bei der thermischen Polymerisierung finden sich Proteinmonomere unter Anwendung von höheren Temperaturen zu Aggregaten zusammen. Die thermische Polymerisierung ist am Beispiel von Albuminen in EP-A-269 092 beschrieben.

Die chemische Polymerisierung von Proteinmonomeren erfolgt mit nicht-proteinhaltigen homooder heterobifunktionellen Linkermolekülen. Diese Verfahren zur Vernetzung von Proteinen sind dem Fachmann bekannt und werden beispielsweise in GB-A 1505 400, EP-A-0 122 209 oder EP-A 269 092 beschrieben. Beispiele für die Vernetzung von Albuminmonomeren mit heterobifunktionellen Linkern sind die Reaktion mit Bis(maleinimido)-methylester, Dimethylsuberimidat, Bisuccinimidyl-suberat, Glutardialdehyd, N-Succinimidyl-3-(2-pyridyldithio)propionat, N-5-Azido-2-nitrobenzoylsuccinimid, N-Succinimidyl(4-Jodacetyl)-aminobenzoat oder die Kombination von Maleinimidohexanoyl-N-Hydroxysuccinimidester (MHS) oder Maleinimido-benzoyl-NHS (MBS) und N-Succinimidyl-3-Acetyl-Thiopropionat (SATP). Beispiele fiir homobifunktionelle Linker sind zum Beispiel Diaminohexan, Carbodiimid und andere.

Bevorzugt werden in den erfindungsgemäßes Verfahren Albuminpolymere, insbesondere Serumalbuminpolymere verwendet, die thermisch aggregiert wurden. Ganz besonders bevorzugt ist thermisch polymerisiertes Rinderserumalbumin ("Thermo-RSA"), das dann anschließend acyliert, insbesondere acetyliert oder succinyliert wird. Die Darstellung des nichtacylierten Thermo-Rinderserumalbumin ist in EP-A 269 092 beschrieben.

Vorteilhafterweise wird dabei die Polymerisation so durchgeführt und gesteuert, daß Polyproteinaggregatpartikel einer bestimmten möglichst einheitlichen Größe entstehen. Bevorzugt ist dabei eine Partikelgroße von 10 - 200 nm. ganz besonders vorteilhaft zwischen 20 und 50 nm. Dies entspricht ungefähr einem Molekulargewicht von 240.000 Da - 2,2 x 10⁹ Da, bevorzugt 2,2 x 10⁶ - 35 x 10⁶ Da. Die Partikelgröße kann über bekannte Verfahren wie z.B. PCS (photon correlation spectroscopy) bestimmt werden. Wenn nötig kann auch der fiir die Erfindung besonders geeignete Partikelgrößenbereich durch Gelfiltration von einem Rohpolymerisatgemisch abgetrennt werden, um eine besonders einheitliche Partikelgröße zu erhalten.

Die Proteinmonomere, die zur Polymerisierung eingesetzt werden, können dabei gleich oder verschieden sein. Bevorzugt werden einheitliche Proteinmonomere polymerisiert. Besonders geeignet sind Albumine. Als Albuminmonomere konnen alle tierischen oder menschlichen Albumine, insbesondere Serumalbumine eingesetzt werden. Ganz besonders fiir die Erfindung geeignet ist Rinderserumalbumin (RSA)

Die Proteinaggregate sind erfindungsgemäß mit -CO-R-Gruppen acyliert, in denen R einen verzweigten oder unverzweigten C1-C4-Alkylrest darstellt, der mit Carboxy, Hydroxy, PO₃H₂ oder SO₃H substituiert sein kann. Bevorzugt als Substituent ist dabei die Carboxy-Gruppe.

Dabei können die Acylgruppen schon in die Proteinmonomere oder erst nach Polymerisation der Proteinmonomere in die Proteinaggregate eingeführt werden. Die Acylierung des Proteins erfolgt dabei mit bekannten Methoden, bevorzugt mit Acylanhydriden oder mit Acyl-O-Succinimid. Als besonders vorteilhaft haben sich acetylierte und succinylierte Proteinaggregate, insbesondere Albuminaggregate erwiesen (R= Methyl bzw. -CH2-CH2-COOH). Dabei wird für die Acetylierung bevorzugt Essigsaure-O-Succinimid verwendet. Die Succinylierung erfolgt bevorzugt mit Bernsteinsaureanhydrid.

Bei der Acylierung werden im wesentlichen freie Aminogruppen (z.B. Lysinreste) des Proteinaggregates acyliert. Unter acylierten Albuminaggregaten wird verstanden, daß mindestens eine der vorhandenen freien Aminogruppen acyliert vorliegt.

Ein weiterer Gegenstand der Erfindung ist ein Entstörmittel für immunologische Teste, enthaltend einen Puffer fiir immunologische Teste und die erfindungsgemäße Entstörsubstanz. Dabei können als Puffer alle waßrige Puffer dienen, die in immunologischen Testen gebräuchlich sind, zum Beispiel Phosphat-, Glycin-HCl Acetat, Carbonat; Citrat-, oder organische Puffer wie z.B. Imidazol, Triethanolamin; MES =(4-Morpholinoethansulfonsäure), TRIS = (TRIS (hydroxymethyl)-aminomethan), HEPES=(4-(2-Hydroxyethyl)-1-piperazinethan-sulfonsäure), MOPS (B-N-Morpholino-propan-sulfonsaure) und andere Puffer. Der pH-Wert und, die Konzentration der Puffersalze richtet sich nach dem jeweiligen immunologischen Test, zum Beispiel unter anderem auch nach dem Enzym bei Enzymmarkierung. Übliche pH-Werte liegen zwischen 4 und 9. Übliche Pufferkonzentrationen liegen zwischen 1 mM und 1M

Die Konzentration an erfindungsgemaßer Substanz zur Signalsteigerung richtet sich danach, mit welcher Menge an Probenflüssigkeit und immunologischen Testkomponenten und den darin enthaltenen Störkomponenten das Mittel zusammen gebracht werden soll. Für gebräuchliche Immunoassays sollte dabei die Konzentration an erfindungsgemäßen acylierten Proteinaggregaten im Mittel so hoch sein, daß nach dem Zusammenbringen mit den immunologischen Testkomponenten, insbesondere mit dem unmarkierten Bindungspartner, eine Konzentration zwischen 1 mg / ml und 50 mg / ml, bevorzugt zwischen 5 und 20 mg / ml resultiert. In Einzelfällen konnen aber auch bis zu 200 mg / ml erforderlich sein.

Zusätzlich können im erfindungsgemäßen Mittel noch weitere Zusätze wie Stabilisierungsmittel und ähnliches anwesend sein. Zum Einsatz in einem Immunoassay liegt das Mittel vorteilhafterweise in wäßriger Pufferlösung vor, zur Lagerung ist aber auch die feste Form, beispielsweise als Lyophilisat oder als Impragnierung eines porösen Trägermaterials (Vlies) z.B. auf einem Teststreifen möglich.

Ein weiterer Gegenstand der Erfindung ist ein spezifisches immunologisches Bindereagenz fiir einen zu bestimmenden Antikörper enthaltend einen spezifischen Bindungspartner des Antikörpers und eine der erfindungsgemaßen Substanzen oder Mittel. Das Bindungsreaganz gemäß der vorliegenden Erfindung wird so hergestellt, daß ein oder mehrere spezifische Bindungspartner eines zu bestimmenden Antikörpers und mindestens eine der erfindungsgemäßen Substanzen oder Mittel gemischt werden. Gegebenenfalls können weitere Zusätze wie Stabilisierungsmittel oder Konservierungsmittel oder ähnliches zugegeben werden. Die Menge des spezifischen Bindungspartners hangt von der immunologischen Testführung, der Menge des zu bestimmenden Antikorpers, der Art der Markierung und anderen Faktoren ab. Im allgemeinen beträgt die Konzentration 0,1 - 20 µg / ml.

Die Menge an acylierten Proteinaggregaten im Bindungsreagenz liegt vorteilhafterweise zwischen 1 mg / ml und 50 mg / ml, bevorzugt zwischen 5 und 20 mg / ml. In Einzelfällen können aber auch höhere Konzentrationen bis zu 200 mg / ml erforderlich sein.

Das spezifische Bindereagenz kann in jedem homogenen oder heterogenen, bevorzugt heterogenen Immunoassay verwendet werden, in dem ein spezifischer Bindungspartner für den Nachweis oder das Fehlen eines spezifisch bindenden Antikörpers nützlich ist. Beispiele dafür sind Sandwichassays, kompetitive Immunoassays und andere dem Fachmann bekannte Immunoassays. Die Teste können in Losung oder auf festen Trägern durchgeführt werden.

Im allgemeinen wird das erfindungsgemäße Immunoassayverfahren so durchgeführt, daß eine einen Antikörper enthaltende Probe mit einem spezifischen Bindungsreagenz gemäß der vorliegenden Erfindung in Lösung kontaktiert wird, so daß sich ein spezifischer Bindekomplex direkt oder indirekt zwischen dem Antikörper und dem spezifischen Bindungspartner bildet. Der Antikörper und der Bindungspartner können direkt komplexieren. Der Bindungspartner ist dann spezifisch für den Bindungspartner. Möglich ist aber auch, daß der Bindungspartner mit dem Liganden über eine oder mehrere spezifische Bindemoleküle komplexiert, die mit dem Antikörper untereinander binden.

Wird das Verfahren auf festen Tragern, zum Beispiel Tubes, Mikrotiterplatten oder einem Testträger als heterogener Immunoassay eingesetzt, kann auf dem Träger ein spezifischer Bindungspartner für den Antikörper direkt immobilisiert sein. Bevorzugt ist aber, daß auf dem Träger ein spezifischer Bindungspartner für den Antikörperbindungspartner immobilisiert ist. Ein bevorzugtes Beispiel dafür ist immobilisiertes Streptavidin als spezifisches Bindereagenz für biotinylierte Bindepartner. Die verschiedenen Reaktionen solcher heterogener Immunoassays sind dem Fachmann bekannt.

Bei kompetitiven Immunoassays konkurrieren Antikorper und ein markiertes Antikörperanalogon oder ein markiertes Antikörperfragment um den unmarkierten Ligandenbindungspartner, der sich - bei heterogenen Immunoassays - über eine zweite Bindungsstelle (bevorzugt eine spezifische Bindestelle wie Biotin) an die Festphase binden kann. Freie oder gebundene Antikorperanaloge werden durch ihre Markierung als Maß für die Anwesenheit oder Menge des zu bestimmenden Antikörpers gemessen.

Bei Sandwichimmunoassays bindet der Antikörper mit einer ersten spezifischen Bindungsstelle an einen markierten Bindungspartner und mit der zweiten Bindungsstelle an einen unmarkierten, - bei heterogenen Immunoassays - immobilisierten oder mit einer weiteren spezifischen Bindungsstelle für die Festphase versehenen Antikörperbindungspartner. Es bildet sich ein Komplex zwischen Antikörper, markiertem und unmarkiertem Bindungspartner, wobei sich der Komplex bei heterogenen Testen über den unmarkierten Bindungspartner an die Festphase bindet, und beispielsweise durch Waschen von dem freien markierten Bindungspartner getrennt werden kann. Bestimmt wird das Signal von freien oder gebundenen markierten Antikörperbindungspartnern als Maß für die Anwesenheit oder Menge des zu bestimmenden Antikörpers nach bekannten Methoden. Bei Enzymmarkierung beispielsweise wird zum markierten Spezies ein farbbildendes Enzymsubstrat gemessen und die entstandene Farbe als Signal gemessen.

Die erfindungsgemäßen Substanzen zeigen besonders vorteilhafte Wirkung in Sandwichimmunoassays, insbesondere heterogenen Sandwichimmunoassays.

Als ganz besonders vorteilhaft für die Wirkung der erfindungsgemaßen Substanzen hat es sich herausgestellt, wenn die Probe mit dem unmarkierten Bindungspartner zusammen mit einem erfindungsgemäßen acylierten Albuminaggregat in Lösung inkubiert wird und erst dann der markierte Bindungspartner zu dieser Lösung zugegeben wird.

Ein weiterer Gegenstand der Erfindung ist die Verwendung der erfindungsgemäßen Substanzen in Immunoassays. Insbesondere ist Gegenstand der Erfindung die Verwendung der erfindungsgemäßen Substanzen zur Steigerung der Signalintensität und zur Reduktion falsch-negativer Analyseergebnisse in Immunoassays. Es hat sich gezeigt, daß bei Einsatz der erfindungsgemäßen Substanzen oder Mittel in einem Immunoassay auf Antikörper ansonsten kaum meßbare Signale bei niederen Antikorperkonzentrationen beträchtlich verstärkt werden. Die Empfindlichkeit des Immunoassay wird so beträchtlich gesteigert, daß Proben, die ohne die erfindungsgemäßen Substanzen als negativ bewertet wurden, deutlich als Antikörper-positiv identifiziert werden konnen. Der Signalanstieg kann bis zum 2 - 5-fachen betragen. Ein solch drastischer Anstieg des Signals bei Antikorpertesten durch Einsatz der erfindungsgemäßen polymeren Substanzen war insofern auch überraschend, da durch die Anwesenheit der erfindungsgemäßen Substanzen die Viskosität in der Inkubationslösung steigt, dadurch die Diffusion der Antiköper und Antikörperantigenkomplexe zur Wand vermindert und so eher ein erniedrigtes Signal im Test erwartet werden sollte.

Ein weiterer Gegenstand der Erfindung ist ein Verfahren zur Herstellung der erfindungsgemäßen Entstorsubstanzen. Es ist dadurch gekennzeichnet, daß bevorzugt in einem ersten Schritt ein Protein, bevorzugt ein Albumin, bevorzugt Rinderserumalbumin, durch chemische Aggregation mit bifunktionellen Linkern, bevorzugt durch thermische Aggregation aggregiert wird, bevorzugt auf eine Partikelgröße zwischen 10 und 200 nm, ganz besonders bevorzugt zwischen 20 und 50 nm. Die thermische Aggregation erfolgt bevorzugt bei einer Temperatur zwischen 50 und 100 °C, ganz besonders bevorzugt zwischen 60 und 80 °C. In einem zweiten Schritt erfolgt dann die Acylierung mit einer -CO-R-Gruppe mit einem geeigneten Acylierungsmittel. Die Acylierung soll dabei bevorzugt vollständig verlaufen und kann über den Verbrauch an Acylierungsmittel uber beispielsweise HPLC mitverfolgt werden.

Es ist jedoch auch möglich, das Verfahren in umgekehrter Reihenfolge durch Acylierung des Proteins gemäß US-A-5,051,356 und anschließender thermischer oder chemischer Polymerisation des acylierten Proteins durchzuführen.

### Beispiel 1

Einfluß von acetyliertem Thermo-RSA auf die Signale von HCV positiven Seren

Sandwichimmunoassay auf Anti-HCV mit Enzymun ® der Firma Boehringer Mannheim:
1. Inkubarionspuffer:
   Natriumphosphat 40 mmol /1 pH 7,4
   Natriumchlorid 7.1 g / l
   N-Methylisothiazolon-HCl 1g / l
   2-Chloracetamid 1 g / l
   Plasma-Diagnostic-based 210 ml / l
   HCV-Peptide biotinyliert (5 - 100 ng / 1, je nach Antigen)
   Thermo-RSA acetyliert in verschiedenen Konzentrationen zwischen 0 und 1 % (0,1 % = 1mg/ml), Partikelgröße 30 nm
2. Konjugatpufferlösung
   Phosphatpuffer 40 mmol / l pH 7.0
   Rinderalbumin 1 g / l
   Rinder IgG 4 g / l
   Triton X 100 1 g / l
   Anti-Human-Fcγ-Antikörper (Schaf) -POD
3. Substratpuffer:
   Phosphat/Citrat 10 mmol / l, pH 4,4
   H₂O₂ 3,2 mmol / l, ABTS 1,2 mmol / l

Die Testdurchführung mit verschiedenen Proben 1-4 geschieht auf ES 600 der Firma Boehringer Mannheim GmbH
1. Schritt: 1 Stunde. Inkubation von 20 µl Probe + 0,5 ml Inkubationspuffer in einem streptavidinbeschichteten Tube
2. Schritt: 1 Stunde: Zugabe von 0,5 ml Konjugatpuffer mit Konjugat Waschschritt
3. Schritt: 1 Stunde Inkubation mit 0.5 ml Substratpuffer mit Chromogen

### Messung der Substratlösung bei 422 nm

Tabelle 1 gibt den Einfluß steigender Konzentrationen an acetylierten Thermo-RSA auf die Meßwerte der Proben 1 - 4 an. Die Messwertsignale werden mit steigender Konzentration an acetyliertem Thermo-RSA bei gleichbleibendem Nullwert deutlich hoher. Daraus resultiert eine deutlich höhere Empfindlichkeit.

### Signale am ES 600 bei 422 nm in Extinktionen

| **Proben** | **Zusatz von TRSA-Acetyliert im Inkubationspuffer** | | | | | | |
|---|---|---|---|---|---|---|---|
| | **0%** | **0,10%** | **0,20%** | **0,30%** | **0,50%** | **1%** | **2%** |
| **Serum 1** | 0,294 | 0,414 | 0,475 | 0,684 | 0,689 | 0,805 | 1,108 |
| **Serum 2** | 1,390 | 1,735 | 2,247 | 2,360 | 2,440 | 2,820 | 2,921 |
| **Serum 3** | 0,369 | 0,562 | 0,608 | 0,709 | 0,859 | 1,026 | 1,032 |
| **Serum 4** | 0,260 | 0,387 | 0,452 | 0,596 | 0,656 | 0,723 | 0,889 |
| **Serum 5** | 0,172 | 0,275 | 0,310 | 0,390 | 0,479 | 0,553 | 0,580 |
| **Serum 6** | 0,596 | 0,894 | 1,038 | 1,092 | 1,350 | 1,287 | 1,563 |

### Beispiel 2

Anti HCV-Test mit acetyliertem Thermo-RSA unterschiedlicher Partikeigröße

Der Test wird wie in Beispiel 1 durchgeführt, jedoch befindet sich im Inkubationspuffer 5 mg /ml acetyliertes Thermo-RSA unterschiedlicher Partikelgröße zwischen 25 nm und 85 nm.

### Die Ergebnisse zeigt Tabelle 2

Während bei HCV-negativen Seren kein Signalanstieg zu beobachten ist, wird durch den Einsatz von acetyliertem Thermo-RSA bei unterschiedlicher Partikelgröße ein deutlicher Signalanstieg beobachtet.

### Signale am ES 600 bei 422 nm in Extinktionen

| **Proben** | **Zusatz von TRSA-Acetyliert im Inkubationspuffer** | | | | | |
|---|---|---|---|---|---|---|
| | **ohne** | **25 nm** | **39 nm** | **59 nm** | **66 nm** | **85 nm** |
| **Pos. Serum A** | 0,276 | 0,561 | 0,706 | 0,750 | 0,699 | 0,702 |
| **Pos. Serum B** | 0,150 | 0,332 | 0,357 | 0,409 | 0,413 | 0,398 |
| **Pos. Serum C** | 0,379 | 0,996 | 1,306 | 1,221 | 1,006 | 1,024 |
| **Pos. Serum D** | 0,432 | 1,094 | 1,249 | 1,292 | 1,158 | 1,119 |
| **Negativserum 1** | 0,043 | 0,045 | 0,046 | 0,047 | 0,049 | 0,046 |
| **Negativserum 2** | 0,043 | 0,041 | 0,043 | 0,044 | 0,044 | 0,046 |
| **Negativserum 3** | 0,041 | 0,042 | 0,046 | 0,048 | 0,047 | 0,045 |
| **Negativserum 4** | 0,042 | 0,043 | 0,045 | 0,046 | 0,044 | 0,043 |
| **Negativserum 5** | 0,038 | 0,041 | 0,041 | 0,040 | 0,042 | 0,041 |

### Beispiel 3.

Wie Beispiel 2, jedoch mit succinyliertem Thermo-RSA unterschiedlicher Partikelgröße in Inkubationspuffer.

### Signale am ES 600 bei 422 nm in Extinktionen

| **Proben** | **Zugabe von TRSA-Succinyliert im Inkubationspuffer** | | | | | |
|---|---|---|---|---|---|---|
| | **ohne** | **8 nm** | **18 nm** | **30 nm** | **36 nm** | **74 nm** |
| **Pos. Serum A** | 0,256 | 0,240 | 0,364 | 0,652 | 0,570 | 0,593 |
| **Pos. Serum B** | 0,083 | 0,080 | 0,178 | 0,216 | 0,210 | 0,260 |
| **Pos. Serum E** | 0,407 | 0,330 | 0,619 | 0,858 | 0,797 | 1,009 |
| **Pos. Serum F** | 0,357 | 0,389 | 0,593 | 0,864 | 0,753 | 1,059 |
| **Negativserum 6** | 0,010 | 0,013 | 0,013 | 0,022 | 0,022 | 0,021 |
| **Negativserum 7** | 0,012 | 0,017 | 0,018 | 0,027 | 0,026 | 0,028 |
| **Negativserum 8** | 0,011 | 0,019 | 0,017 | 0,029 | 0,027 | 0,034 |
| **Negativserum 9** | 0,010 | 0,015 | 0,015 | 0,021 | 0,024 | 0,026 |
| **Negativserum 10** | 0,013 | 0,018 | 0,018 | 0,033 | 0,027 | 0,035 |

### Beispiel 4

Herstellung von acetyliertem thermisch aggregiertem Rinderserumalbumin (acetyliertes Thermo-RSA)

### 1. Herstellung von thermovernetztem RSA

1 g RSA wird in 100 ml 50 mM Kaliumphosphatpufferlosung (pH 7,0) auf 70°C erwärmt und bei dieser Temperatur für 4 Stunden gehalten. Dann wird die Losung gekühlt, filtriert und in einer Ultrazentrifuge (Ausschlußgrenze 30.000 Da) auf eine Konzentration von 50 mg/ml gebracht. Dann wird gegen das 30-fache Volumen doppelt destilliertes Wasser dialysiert und anschließend lyophilisiert. Das Produkt hat ein Molekulargewicht von ca 700. 000 und eine Partikelgröße von 30+/- 8 nm (gemessen über Photon Correlation Spectroskopy (PCS)).

### 2. Acetylierung von Thermo-RSA

4000 g thermisch aggregiertes RSA in 100 mM Kaliumphosphatpuffer pH 8,0 wird auf 25°C temperiert. Die Proteinkonzentration wird mittels OD 280 nm bestimmt und danach eine Proteinkonzentration von 10 mg / ml eingestellt. Gegebenenfalls wird mit 10 mM Kaliumphosphatpuffer pH 8,0 korrigiert. Die Thermo-RSA-Lösung im Rührkessel wird auf 25° C erwärmt. Essigsäure-N-Hydroxysuccinimid-Ester wird in einer Konzentration von 100 mg / ml in wasserfreiem DMSO bei Raumtemperatur gelöst. Pro Liter zu acetylierender Thermo-RSA-Losung werden 11,5 ml Succinimidester-Lösung zugegeben. Die damit erreichte Endkonzentration im DMSO betragt ca. 1%. Der Acetylierungsansatz wird dann nach Kontrolle des pH-Wertes (Soll 6,5 - 9) bei 25° C für 120 min gerührt. Die Abnahme des Essigsäure-N-Hydroxysuccinimid-Esters wird über TSK 3000 / HPLC (Detektion 260 nm) verfolgt. Nach der Inkubation wird die Acetylierung durch Zugabe von Lysinhydrochloridlösung auf 5 mM Endkonzentration gestoppt.

Der gestoppte Acetylierungsansatz wird uber eine Filterpresse filtriert. Die Presse wird mit Wasser nachgewaschen Filtrat und Nachwaschwert werden vereinigt.

Das vereinigte Filtrat wird über eine Polysulfonmembran 10 KD auf 50 l konzentriert. Die konzentrierte Lösung wird gegen das 10-fache Volumen 20 mM Kaliumphosphatlösung pH 7,0 diafiltriert. Das Konzentrat wird auf das jeweilige doppelte Volumen mit Diafiltrationspuffer verdünnt und dann wieder auf das Ausgangsvolumen konzentriert. Der Erfolg der Diafiltration wird über TSK 3000 HPLC Analytik ermittelt. Die Lösung wird auf eine Konzentration von 80+- 10 mg / ml konzentriert und im Anschluß daran mit 0.1 % Chloracetamid und 0.01% (Methylisothiazolon) MIT stabilisiert
Die PCS Messung liefert eine Partikelgröße von 30 nm +- 15

### Beispiel 5

### Herstellung von chemisch polymerisiertem, acetylierten Rinderserumalbumin (P-RSA-Succ)

### 1. Vernetzung von Rinderserumalbumin (RSA)

### a. Aktivierung von RSA mit Maleinimidohexanoyl-N-Hydroxysuccinimid (MHS)

3 g RSA werden in 30 ml 30mM Kaliumphosphat-Puffer, pH 7.1, gelöst und mit 0.6 ml einer Lösung aus 180 mg MHS / ml Dimethylsulfoxid (DMSO) versetzt. Nach 1 Std. Inkubation bei 25°C wird der Ansatz ad 10 mM Lysin aufgestockt und gegen das 150-fache Volumen Dialysepuffer (15 mM Kaliumphosphat-Puffer / 50 mM NaCI / 1 mM Ethylendiamintetraacetat (EDTA)/ pH 6.2) dialysiert

### b. Aktivierung von RSA mit S-Acetylthiopropionyl-N-Hydroxysuccinimid (SATP)

3 g RSA werden in 30 ml 30mM Kaliumphosphat-Puffer, pH 7.1, gelöst und mit 0.6 ml einer Lösung aus 140 mg SATP / ml DMSO versetzt. Nach I Std. Inkubation bei 25°C wird der Ansatz ad 10 mM Lysin aufgestockt und gegen das 150-fache Volumen Dialysepuffer (15 mM Kaliumphosphat-Puffer / 50 mM NaCl / 1 mM EDTA/ pH 6.2) dialysiert.

### c. Vernetzung der aktivierten RSA-Komponenten

Die Lösung mit dem SATP-aktivierten RSA aus (b) wird ad 25 mM Hydroxylamin aufgestockt, ein pH von 7.5 eingestellt und 1 Std. bei 25°C inkubiert. Anschließend wird die Lösung mit dem MHS-aktivierten RSA aus (a) zugesetzt und für weitere 45 min bei 25°C inkubiert. Die Vernetzung wird durch Zugabe von 10 mM Cystein gestoppt. Nach weiteren 30 Min wird der Ansatz ad 25 mM N-Methylmaleinimid aufgestockt und gegen das 150-fache Volumen 50 mM Kaliumphosphat-Puffer /0 15 M NaCl/ pH 7.2 dialysiert.

### 2. Succinylierung

Die dialysierte poly-RSA-Lösung aus (1c) wird mit 2.6 ml einer Lösung aus 0.1 g Bernsteinsaureanhydrid / ml DMSO versetzt. Nach Inkubation für 60 Min. bei 25°C wird der Ansatz ad 50 mM Lysin aufgestockt, gegen das 150-fache Volumen 20 mM Kaliumphosphat-Puffer, pH 6.8 dialysiert und lyophilisiert.

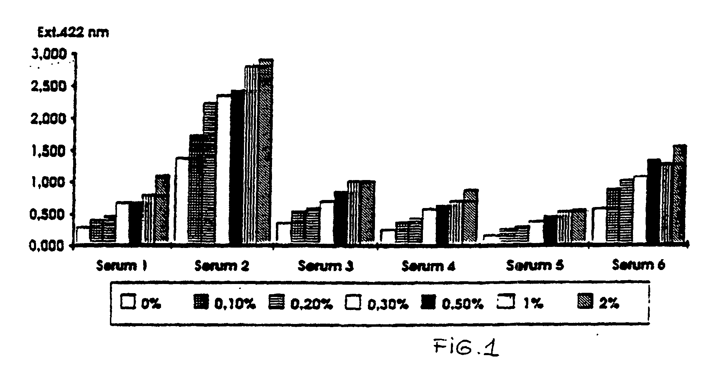

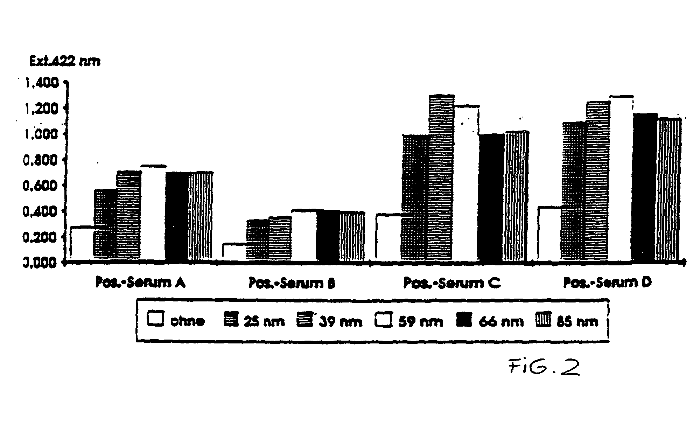

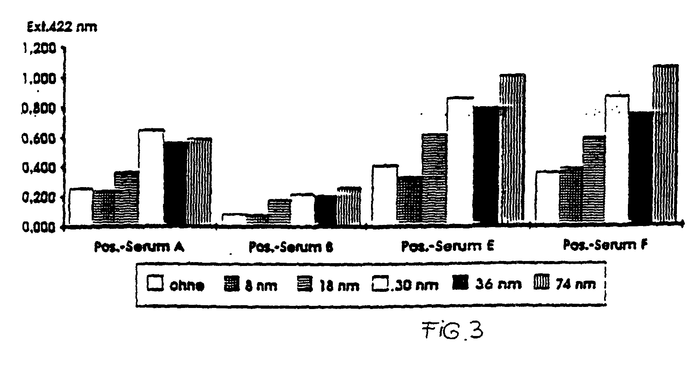

## Patentansprüche

1. Proteinaggregat einer bestimmenten, möglichst einheiteichen größe, das mit - CO-R-Gruppen acyliert ist, wobei R einen verzweigten oder unverzweigten C1-C4 Alkylrest darstellt, der mit Carboxy, Hydroxy, SO₃H oder PO₃H₂ substituiert sein kann.

2. Proteinaggregat gemäß Anspruch 1, **dadurch gekennzeichnet, daß** das Protein ein Albumin ist.

3. Proteinaggregat gemäß Anspruch 2, **dadurch gekennzeichnet, daß** das Albumin Rinderserumalbumin ist

4. Proteinaggregat gemäß den Ansprüchen 1-3, **dadurch gekennzeichnet, daß** R eine Methylgruppe oder eine -CH2CH2-COH-Gruppe darstellt.

5. Proteinaggregat gemäß einem der Ansprüche 1-4, **dadurch gekennzeichnet, daß** das Proteinaggregat chemisch mit homo- oder heterobifunktionellen Linkern aggregiert ist.

6. Proteinaggregat gemäß einem der Anspruche 1-4, **dadurch gekennzeichnet, daß** das Proteinaggregat thermisch aggregiert ist.

7. Proteinaggregat gemäß Anspruch 6, **dadurch gekennzeichnet, daß** es thermisch aggregiertes, acetyliertes oder succinyliertes Rinderserumalbumin darstellt.

8. Proteinaggregat gemäß einem der Anspruche 1-7, **dadurch gekennzeichnet, daß** die Partikelgroße des Aggregates 10 - 200 nm betragt.

9. Proteinaggregat gemäß Anspruch 8, **dadurch gekennzeichnet. daß** die Partikelgröße 20 - 50 nm betragt.

10. Mittel zur Signalsteigerung von Immunoassays zum Nachweis von Antikörpern enthaltend einen Puffer, **dadurch gekennzeichnet, daß** es ein Proteinaggregat gemäß einem der Anspruche 1- 9 enthalt.

11. Mittel gemäß Anspruch 10, **dadurch gekennzeichnet, daß** der Puffer einen pH-Wert zwischen 4 und 9 hat.

12. Spezifisches Bindungsreagenz fiir Immunoassays zum Nachweis eines Antikörpers enthaltend einen oder mehrere spezifische Bindungspartner des Antikörpers, **dadurch gekennzeichnet, daß** es weiterhin ein acyliertes Proteinaggregat gemäß einem der Anspruche 1 - 9 oder ein Mittel gemaß einem der Anspruche 10 oder 11 enthält.

13. Spezifisches Bindungsreagenz gemäß Anspruch 12, **dadurch gekennzeichnet, daß** der Bindungspartner ein biotinylierter Bindungspartner ist.

14. Spezifisches Bindungsregaenz gemäß Anspruch 12 oder 13, **dadurch gekennzeichnet, daß** der Bindungspartner ein Antigen ist.

15. Spezifisches Bindungsreagenz gemäß Anspruch 14, **dadurch gekennzeichnet, daß** ein Bindungspartner ein markierter Anti-Antikörper ist

16. Verfahren zur Bestimmung eines Antikörpers in einer Probe unter Vermeidung einer falsch-negativen Bestimmung durch Steigerung des Meßsignals durch
1) Kontaktieren der auf den Antikörper zu untersuchenden Probe mit einem oder mehreren spezifischen Bindungspartnern des Antikörpers, wobei mindestens ein Bindungspartner markiert ist und mit dem Antikörper ein detektierbares Bindungspaar bildet
2) Messung des Signals des markierten Bindungspaares oder des freien markierten Bindungspartners als Maß für die Anwesenheit oder die Konzentration des Antikörpers in der Probe,
**dadurch gekennzeichnet, daß** die Probe und mindestens einer der spezifischen Bindungspartner mit einem acyliertem Proteinaggregat gemäß einem der Ansprüche 1-9 kontaktiert wird

17. Verfahren gemäß Anspruch 16, **dadurch gekennzeichnet, daß** der Antikörper gegen einen Virus gerichtet ist

18. Verfahren gemäß einem der Anspruche 16 oder 17, **dadurch gekennzeichnet, daß** ein unmarkierter Bindungspartner ein Antigen oder Epitop, das mit dem zu bestimmenden Antikörpers bindungsfähig ist

19. Verfahren gemäß einem der Anspruche 16 - 18, **dadurch gekennzeichnet, daß** der markierte Bindungspartner ein Anti-Antikörper ist.

20. Verfahren gemäß einem der Anspruche 16 -19, **dadurch gekennzeichnet, daß** zuerst Probe und unmarkierter Bindungspartner mit einem acyliertem Proteinaggregat kontaktiert werden und anschließend markierter Bindungspartner zugegeben wird.

21. Verfahren gemaß einem der Anspruche 16 - 20, **dadurch gekennzeichnet, daß** der unmarkierte Bindungspartner eine weitere spezifische Bindungsstelle für einen festphasengebundenen Bindungspartner besitzt

22. Verfahren gemäß Anspruch 21, **dadurch gekennzeichnet, daß** der unmarkierte Bindungspartner biotinyliert ist.

23. Verwendung der acylierten Proteinaugregate gemaß einem der Ansprüche 1-9 fiir Immunoassays.

24. Verwendung der acylierten Proteinaggregate gemäß einem der Ansprüche 1-9 zur Steigerung der Signalintensität und zur Vermeidung falsch-negativer Messergebnisse in Immunoassays.

25. Verfahren zur Herstellung eines acylierten Proteinaggregates gemäß Anspruch 1, **dadurch gekennzeichnet, daß**
a) Proteinmonomere zu einem Aggregat polymerisiert werden.
b) daß das Proteinaggregat mit einem entsprechenden Acylierungsmittel acyliert wird.

26. Verfahren gemäß Anspruch 25, **dadurch gekennzeichnet, daß** die Polymerisierung des Proteins thermisch erfolgt.

27. Verfahren gemaß Anspruch 25, **dadurch gekennzeichnet, daß** das Proteinaggregat mit Acetyl-O-Succinimid acetyliert oder mit Bernsteinsaureanhydrid succinyliert wird.

## Claims

1. Protein aggregate of a particular uniform size which is acylated with -CO-R groups, wherein R is a branched or unbranched C1-C4 alkyl residue which can be substituted with carboxy, hydroxy, SO₃H or PO₃H2 groups.

2. Protein aggregate according to claim 1, **characterized in that** the protein is albumin.

3. Protein aggregate according to claim 2, **characterized in that** the albumin is bovine serum albumin.

4. Protein aggregate according to claims 1 to 3, **characterized in that** R is a methyl group or a -CH₂CH₂-COOH group.

5. Protein aggregate according to one of the claims 1 - 4, **characterized in that** the protein aggregate is chemically aggregated with homo- or heterobifunctional linkers.

6. Protein aggregate according to one of the claims 1 - 4, **characterized in that** the protein aggregate is thermally aggregated.

7. Protein aggregate according to claim 6, **characterized in that** the protein aggregate is thermally aggregated acetylated or succinylated bovine serum albumin.

8. Protein aggregate according to one of the claims 1 - 7, **characterized in that** the particle size of the acylated protein aggregate ranges between 10 and 200 nm.

9. Protein aggregate according to claim 8, **characterized in that** the particle size ranges between 20 - 50 nm.

10. Means for increasing the signal in immunoassays for the detection of antibodies containing a buffer, **characterized in that** it comprises a protein aggregate according to one of the claims 1 - 9.

11. Means according to claim 10, **characterized in that** the buffer has a pH value between 4 and 9.

12. Specific binding reagent for immunoassays for the detection of antibodies comprising one or several specific binding partners of the antibody, **characterized in that** it also comprises an acylated protein aggregate according to one of the claims 1 - 9 or a means according to one of the claims 10 or 11.

13. Specific binding reagent according to claim 12, **characterized in that** the binding partner is a labeled or biotinylated binding partner.

14. Specific binding reagent according to claim 12 or 13, **characterized in that** the binding partner is an antigen.

15. Specific binding reagent according to claim 14, **characterized in that** the binding partner is a labeled antibody.

16. Method for determining an antibody in a sample while avoiding false negative results due to increase in the signal intensity by means of
1. contacting sample to be tested for the antibody with one of several specific binding partners of the antibody, wherein at least one binding partner is labeled, forming a detectable binding pair together with the antibody,
2. measuring the signal of the labeled binding pair or the free labeled binding partner as a measure for the presence or concentration of antibody in the sample,
**characterized in that** the sample and at least one of the specific binding partners is brought into contact with an acylated protein aggregate according to one of the claims 1-9.

17. Method according to claim 16, **characterized in that** the antibody is directed against a virus.

18. Method according to claim 16 or 17, **characterized in that** an unlabeled binding partner is an antigen or epitope capable of binding to the antibody to be determined.

19. Method according to claim 16 - 18, **characterized in that** the labeled binding partner is an anti-antibody.

20. Method according to one of the claims 16 - 19, **characterized in that** the sample and the unlabeled binding partner are first brought into contact with an acylated protein aggregate and the labeled binding partner is subsequently added.

21. Method according to one of the claims 16 - 20, **characterized in that** thre unlabeled binding partner has another binding site for a solid phase-bound binding partner.

22. Method according to claim 21, **characterized in that** the unlabeled binding partner is biotinylated.

23. Use of the acylated protein aggregate according to one of the claims 1-9 in immunoassays.

24. Use of the acylated protein aggregate according to one of the claims 1-9 to increase the intensity of the signal and avoid false-negative measurements in immunoassays

25. Method for preparing an acylated protein aggregate according to claim 1, **characterized in that**
a) protein monomers are polymerized to form an aggregate,
b) the protein aggregate is acylated with a corresponding acylating agent.

26. Method according to claim 25, **characterized in that** the protein is thermally polymerized.

27. Method according to claim 25, **characterized in that** the protein aggregate is acetylated with acetyl-O-succinimide or succinylated with succinic acid anhydride.

## Revendications

1. Agrégat de protéine ayant une taille déterminée, aussi homogène que possible, qui est acylé à l'aide de groupes -CO-R-, où R représente un groupe alkyle en C₁-C₄ linéaire ou ramifié, qui peut être éventuellement substitué par un groupe carboxy, hydroxy, SO₃H ou PO₃H₂.

2. Agrégat de protéine selon la revendication 1, **caractérisé en ce que** la protéine est une albumine.

3. Agrégat de protéine selon la revendication 2, **caractérisé en ce que** l'albumine est de l'albumine de sérum bovin.

4. Agrégat de protéine selon les revendications 1-3, **caractérisé en ce que** R représente un groupe méthyle ou un groupe -CH₂-CH₂-COH.

5. Agrégat de protéine selon l'une quelconque des revendications 1-4, **caractérisé en ce que** l'agrégat de protéine est agrégé chimiquement à l'aide de linkers homo- ou hétérobifonctionnels.

6. Agrégat de protéine selon l'une quelconque des revendications 1-4, **caractérisé en ce que** l'agrégat de protéine est agrégé thermiquement.

7. Agrégat de protéine selon la revendication 6, **caractérisé en ce qu'**il représente de l'albumine de sérum bovin agrégé thermiquement, acétylé ou succinylé.

8. Agrégat de protéine selon l'une quelconque des revendications 1-7, **caractérisé en ce que** la taille des particules de l'agrégat est de 10-200 nm.

9. Agrégat de protéine selon la revendication 8, **caractérisé en ce que** la taille des particules est de 20-50 nm.

10. Agent de renforcement de signal pour un dosage immunologique destiné à la détection d'anticorps contenant un tampon, **caractérisé en ce qu'**il contient un agrégat de protéine selon l'une quelconque des revendications 1-9.

11. Agent selon la revendication 10, **caractérisé en ce que** le pH du tampon est compris entre 4 et 9.

12. Réactif de liaison spécifique pour un dosage immunologique destiné à la détection d'un anticorps, contenant un où plusieurs partenaires de liaison spécifique de l'anticorps, **caractérisé en ce qu'**il contient en outre un agrégat de protéine acylé selon l'une quelconque des revendications 1-9 ou un agent selon l'une des revendications 10 ou 11.

13. Réactif de liaison spécifique selon la revendication 12, **caractérisé en ce que** le partenaire de liaison est un partenaire de liaison biotinylé.

14. Réactif de liaison spécifique selon la revendication 12 ou 13, **caractérisé en ce que** le partenaire de liaison est un antigène.

15. Réactif de liaison spécifique selon la revendication 14, **caractérisé en ce qu'**un des partenaires de liaison est un anti-anticorps marqué.

16. Procédé de détermination d'un anticorps dans un échantillon permettant d'éviter une détermination fausse négative au moyen d'un renforcement du signal de mesure par
1) mise en contact de l'échantillon dans lequel l'anticorps doit être déterminé avec un ou plusieurs partenaires de liaison spécifique de l'anticorps, au moins l'un des partenaires de liaison étant marqué et formant avec l'anticorps un couple de liaison détectable,
2) mesure du signal du couple de liaison marqué ou du couple de liaison marqué libre comme mesure de la présence ou de la concentration de l'anticorps dans l'échantillon,
**caractérisé en ce que** l'échantillon et au moins l'un des partenaires de liaison spécifique sont mis en contact avec un agrégat de protéine acylé, selon l'une quelconque des revendications 1-9.

17. Procédé selon la revendication 16, **caractérisé en ce que** l'anticorps est dirigé contre un virus.

18. Procédé selon l'une des revendications 16 ou 17, **caractérisé en ce qu'**un partenaire de liaison non marqué est un antigène ou un épitope qui peut entrer en liaison avec l'anticorps à déterminer.

19. Procédé selon l'une quelconque des revendications 16-18, **caractérisé en ce que** le partenaire de liaison marqué est un anti-anticorps.

20. Procédé selon l'une quelconque des revendications 16-19, **caractérisé en ce que** d'abord on met en contact l'échantillon et le partenaire de liaison non marqué avec un agrégat de protéine acylé et ensuite, on ajoute le partenaire de liaison marqué.

21. Procédé selon l'une quelconque des revendications 16-20, **caractérisé en ce que** le partenaire de liaison non marqué présente un autre site de liaison spécifique pour un partenaire de liaison lié à la phase solide.

22. Procédé selon la revendication 21, **caractérisé en ce que** le partenaire de liaison non marqué est biotinylé.

23. Utilisation d'un agrégat de protéine acylé selon l'une quelconque des revendications 1-9 pour des dosages immunologiques.

24. Utilisation de l'agrégat de protéine acylé selon l'une quelconque des revendications 1-9 pour renforcer l'intensité de signal et pour éviter des résultats de mesure fausse négative du dosage immunologique.

25. Procédé de préparation d'un agrégat de protéine acylé selon la revendication 1, **caractérisé en ce que**
a) les monomères de protéine sont polymérisés en un agrégat,
b) l'agrégat de protéine est acylé à l'aide d'un agent d'acylation approprié.

26. Procédé selon la revendication 25, **caractérisé en ce que** la polymérisation de la protéine s'effectue par un procédé thermique.

27. Procédé selon la revendication 25, **caractérisé en ce que** l'agrégat de protéine est acétylé à l'aide d'acétyl-O-succinimide ou succinylé à l'aide de l'anhydride succinique.
